# EUROPÄISCHE PATENTSCHRIFT

(11) **EP 1 962 702 B1**
(45) Veröffentlichungstag und Bekanntmachung des Hinweises auf die Patenterteilung: **27.11.2013**
(21) Anmeldenummer: 06829490.9
(22) Anmeldetag: 11.12.2006
(51) Int. Cl.: A61B 17/221

(54) **Vorrichtung zur Entfernung von Thromben aus Blutgefässen**
Device for the removal of thrombi from blood vessels
Dispositif permettant de supprimer les thrombus de vaisseaux sanguins

(30) Priorität: 12.12.2005 DE 102005059670
(43) Veröffentlichungstag der Anmeldung: 03.09.2008
(73) Patentinhaber: Phenox GmbH, 44801 Bochum (DE)
(72) Erfinder: HANNES, Ralf, 44137 Dortmund (DE); SCHNEIDER, Manuel, 45529 Hattingen (DE); PRACHT, Holger, 44649 Herne (DE); MILOSLAVSKI, Elina, 44797 Bochum (DE); MONSTADT, Hermann, 44797 Bochum (DE)
(74) Vertreter: Thiel, Christian
(86) Internationale Anmeldenummer: PCT/EP2006/011900
(87) Internationale Veröffentlichungsnummer: WO 2007/068424

(56) Entgegenhaltungen:
- WO-A-2006/021407
- DE-A1- 3 921 071
- US-A- 5 899 850
- US-A1- 2002 072 764

## Beschreibung

Die Erfindung betrifft eine Vorrichtung zur Entfernung von Fremdkörpern und Thromben aus Körperhohlräumen in Blutgefäßen mit mindestens einem Führungsdraht, der ein distales Element aufweist.

Thromboembolische Erkrankungen wie Herzinfarkt, Lungenembolie, periphere Thrombose, Organembolien, etc. werden typischerweise durch einen Thromboembolus (im Folgenden kurz Thrombus), also einen viskoelastischen Blutklumpen aus Blutplättchen, Fibrinogen, Gerinnungsfaktoren etc., ausgelöst, der sich in einem Blutgefäß festgesetzt hat und dieses ganz oder teilweise verschließt. Der Verschluss von Organarterien führt dabei zu einer Unterbrechung der Versorgung des abhängigen Gewebes mit Sauerstoff und Nährstoffen. Der Störung des Funktionsstoffwechsels mit Funktionsverlust folgt innerhalb kurzer Zeit das Erliegen des Strukturstoffwechsels mit dem Untergang des betroffenen Gewebes (Infarkt). Die häufigsten hiervon beim Menschen betroffenen Organe sind das Herz und das Gehirn. Solche Veränderungen betreffen aber auch die Extremitätenarterien und die Lungenarterien. Venöse Thrombosen und thromboembolische Verschlüsse kommen gehäuft in den Bein- und Beckenvenen vor. Das Krankheitsbild des thrombotischen Verschlusses eines intrakraniellen Sinus kann durch die Störung der venösen Drainage des Hirngewebes zu schweren Hirnblutungen führen.

Angesichts der Schwere der durch Thromboembolien ausgelösten Krankheitsbilder und der Häufigkeit dieser Erkrankungen sind verschiedene Techniken zur Auflösung oder Entfernung von Thromben bekannt.

So ist es bekannt, solche Patienten mit thrombolytischen Mitteln wie Streptokinase oder Urokinase oder mit Antikoagulantien zu behandeln, was zur Thrombolyse oder zur Eindämmung des Thrombenwachstums führen soll. Da diese Behandlungsmethoden meist zeitintensiv sind, werden sie oftmals zusammen mit Eingriffen kombiniert, die der mechanischen Zerkleinerung oder Entfernung des Thrombus bzw. Embolus dienen.

Neben offenen chirurgischen Eingriffen kommen im Stand der Technik zunehmend transluminale bzw. endovaskuläre, Katheter geführte interventionelle Therapieformen zum Einsatz, da diese weniger invasiv sind. So ist es bekannt, den Thrombus mittels Unterdruck erzeugenden Saug-Kathetern oder mechanisch mit Kathetern, welche mit Fangkörben, Wendeln, Haken oder dergleichen versehen sind, aus dem Körper des Patienten zu entfernen, siehe US 6 245 089 B1, US 5 171 233 A1, Thomas E. Mayer et al., Stroke 2002 (9), 2232.

Der Nachteil der bekannten transluminalen Vorrichtungen besteht darin, dass auch diese den Thrombus häufig nicht vollständig entfernen können, und die Gefahr besteht, dass der Thrombus oder Fragmente davon sich freisetzen und im Blutstrom zu kleinlumigeren Gefäßen weiterziehen, welche schwerer zu erreichen und zu behandeln sind. Des weiteren eignen sich die im Stande der Technik bekannten Vorrichtungen aufgrund ihrer Dimensionen und/oder geringen Flexibilität nur ungenügend zur Entfernung von Thromben aus besonders kleinlumigen oder stark gewundenen Gefäßen, wie denen des Gehirns.

So ist aus der US 2002/0049452 eine Vorrichtung mit einem Katheter zur Entfernung von Thromben bekannt, an dessen distalem Ende Fangarme aus einem Formgedächtnismaterial angebracht sind, die im komprimierten Zustand am Katheter anliegen und sich in der expandierten Konfiguration radial vom Katheter nach außen erstrecken. Nach der durch die Körpertemperatur ausgelösten Einnahme der expandierten Konfiguration sollen sich die Fangarme im Thrombus verhaken und diesen bei Rückführung des Katheters in einen weiteren Katheter aus dem Blutgefäß mit sich ziehen. Der Nachteil dieser Vorrichtung besteht darin, dass sie entweder zur Kühlung der Fangarme unter die Umwandlungstemperatur, bis diese in den Blutstrom gelangen, in einem Sekundärkatheter, der diese Kühlung ermöglicht, an dem Thrombus vorbei manövriert werden muß; oder der mit den Fangarmen versehene Katheter muss in seinem Inneren eine Heizvorrichtung tragen, die die Erhitzung auf die Umwandlungstemperatur nach dem Erreichen des Thrombus ermöglicht. Diese konstruktiven Erfordernisse sind zum einen sehr aufwendig und somit auch störanfällig und machen allein aufgrund ihres physikalischen Umfanges die Behandlung besonders kleinlumiger Gefäße unmöglich.

Offenlegungsschrift DE 39 21 071 A1 offenbart eine Vorrichtung gemäß dem Obererbegriff des Anspruchs 1. WO 2006/021407 A2 ist nur für die Beurteilung der Neuheit relevant.

Angesichts der mit dem Stand der Technik verbundenen Nachteile besteht die Aufgabe der Erfindung somit in der Bereitstellung einer Vorrichtung zur Entfernung von Fremdkörpern und Thromben aus Körperhohlräumen und Blutgefäßen, welche das operative Risiko bei der Entfernung von Thromben vermindert und die Behandlung besonders kleinlumiger Gefäße erlaubt.

Diese Aufgabe wird erfindungsgemäß gelöst durch eine Vorrichtung zur Entfernung von Fremdkörpern und Thromben aus Körperhohlräumen und Blutgefäßen gemäß Anspruch 1.

Das Grundprinzip der Erfindung besteht darin, eine Vorrichtung zu schaffen, die im Wesentlichen über zwei Elemente verfügt, die durch ihr Zusammenwirken ein sicheres Entfernen von Thromben aus Blutgefäßen ermöglichen. Bei dem einen dieser Elemente handelt es sich um das distale Element, das über radial nach außen hervorstehende Fasern verfügt, während das andere Element die Korb struktur darstellt. Der oder die als Einführhilfe ausgestalteten Führungsdrähte ermögtichen die gute Manövrierbarkeit in auch kleinlumige und gewundene Gefäßabschnitte. Die Fasern des distalen Elements sind geeignet, einen Thrombus festzuhalten und zu stabilisieren, insbesondere dann, wenn sie aus thrombogenem Material bestehen oder damit ausgerüstet sind.

Gemäß der Erfindung ist die selbstexpandierende Drahtstruktur fest mit dem Führungsdraht verbunden und erstreckt sich nur zum Teil über das bürstenartige distale Element. Im Gegensatz zu den anderen Beispielen sind Mikrobürste und Drahtstruktur somit nicht einander gegenüber längs verschieblich. Insgesamt wird auf diese Weise eine besonders kurze Bauform der Vorrichtung realisiert, was hinsichtlich der Handhabung, insbesondere bei engen Blutgefäßen, von Vorteil ist.

Bei Anwendung der Vorrichtung, d. h. bei Rückzug in Richtung proximal, löst die Drahtstruktur den zu entfernenden Thrombus von der Innenwand des Gefäßes und transportiert diesen in Richtung proximal. Das mikrobürstenartige distale Element besitzt die Aufgabe, den Thrombus bzw. seine Fragmente ebenfalls in Richtung proximal zu bewegen. Durch die Selbstexpansion der Drahtstruktur wird die Mikrobürste insbesondere im proximalen Bereich zentriert und kann so ihrer Reinigungs- und Filteraufgabe besonders gut gerecht werden.

Die Drahtstruktur besteht vorteilhafterweise aus einem Drahtgeflecht oder schlaufenförmig verlaufenden Drähten. Ein solches Geflecht bietet den besonderen Vorteil, dass es selbst in äußerst gewundenen Gefäßen formstabil bleibt und sich nicht oval verformt. Auf diese Weise wird sichergestellt, dass die Gefäßinnenwände stets berührt werden. Um die Selbstexpansion der Drahtstruktur bei Ausschieben aus dem Mikrokatheter sicherzustellen, ist es sinnvoll, eine Drahtstruktur zu verwenden, die zumindest teilweise aus einem Formgedächtnismaterial, insbesondere Nitinol, besteht.

Proximal des distalen Elementes läuft die Drahtstruktur zweckmäßigerweise in einem Punkt auf dem Führungsdraht zusammen, wo die Festlegung mittels einer entsprechenden Hülse oder Mikrowendel erfolgen kann. In der Regel ist die Drahtstruktur an ihrem distalen Ende hingegen offen. Die Drähte können insbesondere von proximal zum distalen Ende der Drahtstruktur verlaufen, dort eine Schleife ausbilden und wieder nach proximal zurückverlaufen, so dass die Drahtstruktur selbst keine freien Drahtenden aufweist.

Das distale Element mit seinen radial hervorstehenden Fasern stellt vorzugsweise eine konische Mikrobürste dar, d. h. der Durchmesser des distalen Elements nimmt von proximal nach distal zu, entsprechend der Zunahme der Länge der Fasern. Es hat sich herausgestellt, dass eine solche konische Mikrobürste bei der Beseitigung von Thromben und Thrombusfragmenten besonders effizient ist.

Darüber hinaus sei angemerkt, dass auch gemäß der Erfindung hinsichtlich der verwendeten Materialien für die Fasern und den Führungsdraht, des Aufbaus der Draht bzw. Korbstruktur, der Verbindung der Drähte untereinander bzw. mit dem Führungsdraht etc. das gleiche gilt, wie es zu den anderen Beispielen gesagt wurde.

Zur Behandlung besonders kleinlumiger Gefäße eignen sich insbesondere Fasern einer Länge von 0,5 bis 6 mm und vorzugsweise 1,2 bis 3 mm, so dass auch bei radialer Anordnung der Fasern ein Außendurchmesser des die Fasern tragenden Teils des distalen Elements von 1 bis maximal 12 mm erreicht wird. Zur besonders atraumatischen Behandlung kann dieser Außendurchmesser etwas kleiner dimensioniert sein als der Innendurchmesser des betreffenden Blutgefäßes.

Die Fasern erstrecken sich zweckmäßigerweise über eine Länge des distalen Elements von 0,5 bis 5 mm. Zur Gewährleistung einer ausreichend guten Verankerung des Thrombus ist es zwecksmäßig, wenn die Fasern in einer Dichte von 20 bis 100 pro cm an dem distalen Element des Führungsdrahtes angeordnet sind.

Zweckmäßigerweise ist der Führungsdraht aus einem medizinischen Edelstahl oder einem Formgedächtnismaterial, vorzugsweise Nitinol, hergestellt. Dabei ist es zweckmäßig, wenn der/die Führungsdrähte einen Außendurchmesser von 0,2 bis 0,4, vorzugsweise 0,22 bis 0,27 mm aufweisen. Eine typische Länge eines Führungsdrahtes liegt zwischen 50 und 180 cm.

Gemäß einer weiteren vorteilhaften Ausgestaltung der Vorrichtung sind die Fasern spiralförmig entlang der Längsachse des distalen Elements angeordnet. Diese Ausführungsform eignet sich besonders gut zum "Aufspießen" des Thrombus, da der die Fasern tragende Teil des distalen Elements nach Art eines Korkenziehers arbeitet, wenn er vom behandelnden Arzt entsprechend manövriert wird.

Einer weiteren vorteilhaften Ausführungsform der Erfindung zufolge hat das distale Element mit seinen radial hervorstehenden Fasern nach Ausbringen aus dem Mikrokatheter eine konische Struktur, d: h. die radiale Ausdehnung der Fasern, was letztlich dem Durchmesser des distalen Elements entspricht, nimmt von proximal nach distal zu. Der wesentliche Vorteil einer solchen konischen "Bürstenform" liegt darin, dass unabhängig von der Weite des konkret zu reinigenden Blutgefäßes stets zumindest einige Bereiche vorhanden sind, bei denen die Fasern die optimale Länge aufweisen. Eine optimale Länge für ein bestimmtes Blutgefäß weisen Fasern gerade dann auf, wenn die Fasern an den Wänden des Blutgefäßes anliegen, ohne bei der Bewegung der Vorrichtung in Richtung proximal in distale Richtung verbogen zu werden. In diesem Fall ist die Reinigungswirkung der Fasern besonders gut. Längere Fasern hingegen werden bei der Rückwärtsbewegung in proximaler Richtung in Richtung distal verbogen und reinigen daher nicht mehr effektiv, kurze Fasern wiederum erreichen u. U. gar nicht die Gefäßinnenwand und bewirken daher dort ohnehin keine Reinigungswirkung.

Zusätzlich oder alternativ können auch die Fasern im proximalen Bereich des distalen Elements härter ausgebildet sein als im distalen Bereich. Die härteren Fasern im proximalen Bereich dienen dabei mehr dem Abschaben eines an der Gefäßwand haftenden Thrombus, während die weicheren Fasern im distalen Bereich mehr dem Festhalten des Thrombus bzw. von Thrombusfragmenten dienen.

Die erfindungsgemäß zum Einsatz kommenden Fasern stehen vorzugsweise in einem Winkel von 70° bis 110°, vorzugsweise in einem Winkel von 80° bis 90° von der Längsachse der Vorrichtung ab. Diese Winkelangaben sind so zu verstehen, dass Winkel < 90° eine proximale Ausrichtung der Fasern, Winkel > 90° eine distale Ausrichtung der Fasern bezeichnen. Ausführungsformen mit einem Winkel, der etwas kleiner ist als 90°, sind besonders atraumatisch beim Vorschieben im Gefäß bzw. durch den Thrombus und bewirken gleichzeitig eine besonders gute Verankerung im Thrombus beim Zurückziehen.

Die Fasern können durch Verflechten, Einklemmen, Kleben, Verknoten, Verschweißen und/oder Verschmelzen am distalen Element festgelegt werden. Techniken, um Fasern in dieser Art und Weise zu verbinden, sind beispielsweise aus der Fertigung von faserbewehrten Embolisationsspiralen bekannt.

Gemäß einer besonders bevorzugten Ausführungsform geht man bei der Herstellung des distalen Elements so vor, dass die Fasern nebeneinander und ggf. zusätzlich übereinander zwischen zwei Seelendrähte gelegt werden, wobei die Fasern orthogonal zu den Seelendrähten verlaufen. In diesem Zusammenhang sei angemerkt, dass unter einem orthogonalen Verlauf gemäß der Erfindung nicht lediglich ein Winkel von exakt 90° verstanden wird, sondern jeglicher Querverlauf der Fasern zu den Seelendrähten, d.h. die Fasern verlaufen im Wesentlichen quer zu den Seelendrähten, nicht parallel. Entsprechend können auch Winkel von beispielsweise 70° in diesem Zusammenhang noch als orthogonal aufgefasst werden. Wenn die Fasern zwischen die Seelendrähte eingelegt sind, werden diese miteinander verdrillt, beispielsweise indem ein Ende festgehalten wird, während das andere gedreht bzw. tordiert wird, um eine plastische Verformung der Seelendrähte zu einer Spiralstruktur herbeizuführen. Nach der Verdrillung der Seelendrähte stehen die Fasern gewissermaßen auf einer Schraubenlinie aus den verdrehten Seelendrähten nach außen hervor. Der wesentliche Vorteil eines solchen distalen Elements liegt darin, dass relativ wenig Seelendraht verwendet werden muß, um gleichzeitig einen sehr hohen Faserbesatz zu erreichen. Die Verwendung von Seelendrähten ist insofern vorteilhaft, als das System dadurch besonders flexibel bleibt. Darüber hinaus ist die Festlegung der Fasern an den Seelendrähten in dieser Ausführungsform besonders einfach und die Verteilung der Fasern besonders gleichmäßig.

Die Fasermenge bzw. -dichte läßt sich u.a. durch die Anzahl der Verdrehungen der Seelendrähte steuern, was zu unterschiedlichen Härtegraden bezogen auf die Radialkraft des bürstenartigen distalen Elements führt, da durch mehr Verdrehungen auch mehr Fasern pro Längeneinheit erzeugt werden. Darüber hinaus läßt sich die Biegesteifigkeit u. a. durch die Anzahl der Seelendrähte und der Verdrehungen einstellen.

Ggf. können die Vorrichtungen auch mehrere distale Elemente aufweisen, von denen aus Fasern radial nach außen hervorstehen. Ein solches System kann beispielsweise Vorteile aufweisen, wenn besonders große Thromben oder auch mehrere Thromben aus dem Blutgefäßsystem entfernt werden sollen. Darüber hinaus kann ein weiter distal gelegenes Element mit Faserbesatz ggf. Bruchstücke eines Thrombus, die vom weiter proximal gelegenen distalen Element abfallen, auffangen und mit entfernen.

Um trotz der Länge eines solchen Systems noch eine ausreichende Flexibilität zu erreichen, ist es sinnvoll, die einzelnen distalen Elemente über Verbindungselemente, insbesondere Gelenke, miteinander zu verbinden. An diesem Gelenk kann die Vorrichtung in gewissem Maße abknicken und damit dem Verlauf der Blutgefäße folgen.

Die radial außen liegenden Enden der Fasern weisen vorteilhafterweise Verdickungen auf, beispielsweise kugelförmige Verdickungen, um so der Clotmasse mehr Oberfläche und Halt zur Verfügung zu stellen. Ein weiterer Vorteil dieser Ausführungsform besteht darin, dass die Faserenden auf diese Weise atraumatisch gestaltet werden können. Die Verdickungen an den Faserenden lassen sich beispielsweise dadurch erzielen, dass das Trennen der Fasern mittels Mikrolaserschneiden, Elektronenstrahlschneiden o. ä. erfolgt.

Gemäß einer weiteren Ausführungsform sind die radial außen liegenden Enden der Fasern zumindest teilweise über Schlaufen miteinander verbunden. Letztlich handelt es sich bei den miteinander verbundenen Fasern nicht um zwei, sondern lediglich um eine Faser mit einem schlaufenförmigen Verlauf. Die Faser tritt radial nach außen hervor, verläuft bis zum äußeren Rand des expandierten distalen Elements, bildet eine Schlaufe aus und läuft zurück zum Zentrum des distalen Elements. Insgesamt ergibt sich somit ein elliptischer Verlauf der Fasern. Diese Ausführungsform hat den Vorteil, dass, ähnlich wie bei der Verdickung am Faserende, der Clotmasse mehr Oberfläche zur Verfügung gestellt wird, um auf diese Weise die Einfangwirkung für den Thrombus zu verbessern. Darüber hinaus ist die Schlaufe abgerundet und damit atraumatisch. Ein weiterer Vorteil besteht darin, dass die Fasern steifer werden, da die schlaufenförmig verlaufende Faser letztlich ein Verhalten wie zwei nebeneinander verlaufende Fasern aufzeigt.

Ggf. kann es vorteilhaft sein, wenn die Fasern zumindest teilweise unterschiedlich weit zu den Seiten des distalen Elements radial nach außen hervorstehen. Ähnlich wie bei der zuvor beschriebenen Ausführungsform, bei der die Radialausdehnung des distalen Elements von proximal nach distal zunimmt, kann auch auf diese Weise bewirkt werden, dass stets zumindest einige Fasern vorhanden sind, die die optimale Länge für die Reinigungswirkung aufweisen. Dies kann u. a. erreicht werden, indem der oder die Drähte, von denen die Fasern des distalen Elements ausgehen, außerhalb des Zentrums, d. h. exzentrisch verlaufen. Auf diese Weise wird erreicht, dass auf einer Seite vergleichsweise kurze, auf der anderen Seite vergleichsweise lange Fasern vorhanden sind. Aufgrund ihres kurzen Abstandes zum Befestigungspunkt verhalten sich dabei die kurzen Enden der Fasern deutlich härter, die langen Enden deutlich weicher, wobei wiederum die härteren Fasern eher einer Verbesserung der Reinigungswirkung dienen, die weicheren Fasern eher dem Festhalten des Thrombus. Ein weiterer Vorteil eines distalen Elements mit exzentrisch angeordnetem Drahtverlauf kann darin liegen, dass ein solches distales Element leichter seitlich an einem Clot vorbeigeführt werden kann, um ihn anschließend bei der Rückwärtsbewegung in proximaler Richtung aufzunehmen.

Gemäß einer besonders bevorzugten Ausführungsform der Vorrichtung sind die Fasern beschichtet. Dies kann beispielsweise eine neutrale Beschichtung aus Parylen oder Polytetrafluorethylen (Teflon) sein, aber auch eine solche mit Kollagen oder mit einem die Blutgerinnung förderndem Material, vorzugsweise einem oder mehreren Gerinnungsfaktoren. Diese Ausführungsform dient der verstärkten Verankerung der Fasern im Thrombus und vermindert die Gefahr, dass der Thrombus soweit zerfällt, dass Teile des Thrombus im Blutgefäß zurückbleiben oder sich im Blutstrom freisetzen können.

Es wurde überraschend gefunden, dass eine thrombogene Ausrüstung der Fasern zu einer erheblichen Stabilisierung des Thrombus an der erfindungsgemäßen Vorrichtung führen. Es ist dabei dem Operateur überlassen, die erfindungsgemäße Vorrichtung so mit dem Thrombus in Kontakt zu bringen und zu belassen, dass über eine gewisse Einwirkungszeit der thrombogenen Elemente ein "Festwachsen" des Thrombus an der Vorrichtung gefördert wird. Dieses Festwachsen erfolgt in relativ kurzer Zeit an thrombogenen Fasern, teilweise innerhalb einiger Minuten. Dies beugt nicht nur einem Zerfall des Thrombus vor, wie er mit vielen Vorrichtungen beobachtet wird, sondern erleichtert auch die Einholung des Thrombus in einen Katheter und seine Extraktion aus dem vaskulären System. Die dazu besonders geeigneten thrombogenen Materialien und Beschichtungen sind dem Fachmann aus der Literatur bekannt. Besonders eignen sich etwa einer oder mehrere der Faktoren Fibrin, Thrombin, Faktor XIII und/oder Faktor VIII.

Des Weiteren ist das distale Element mit seinem Faserbesatz etwas länger ausgebildet als die Korbstruktur. Auf diese Weise wird gewährleistet, dass auch nach Einziehen des distalen Elements in die Korbstruktur sich ablösende Thrombusfragmente von den distalen Bereichen des distalen Elements aufgehalten werden. Gewissermaßen wird die Korbstruktur am distalen Ende durch die überstehenden Fasern des distalen Elements verschlossen. Insbesondere dann, wenn der Führungskatheter einen vergleichsweise kleinen Innendurchmesser im Verhältnis zum Außendurchmesser der Vorrichtung aufweist, kann auf diese Weise verhindert werden, dass bei Rückzug in den Führungskatheter ein Herausquetschen der Clotmasse erfolgt.

Vorteilhafterweise weist die Vorrichtung einen oder mehrere radiopake (röntgendichte) Marker auf. Diese können beispielsweise aus Platin oder einer Platinlegierung bestehen. Derartige röntgendichte Marker können sich sowohl im Bereich des distalen Elements als auch im Bereich der Korb-/Röhrenstruktur befinden, so dass der behandelnde Arzt mit den entsprechenden bildgebenden Verfahren die relative Positionierung zueinander und den Fortgang der Behandlung beobachten kann.

Es ist weiterhin vorteilhaft, wenn die Spitze der gesamten Vorrichtung atraumatisch, z. B. abgerundet, ausgebildet ist.

Die Erfindung betrifft schließlich auch die Kombination der Vorrichtung mit einem Führungs- und/oder Mikrokatheter, in dem die Vorrichtung an den Einsatzort manövriert und mit dem Thrombus beladen wieder aus dem Blutgefäßsystem entfernt werden kann. Es kann sinnvoll sein, den Katheter dazu zusätzlich als Aspirationskatheter auszulegen, mit dem Mikrokatheter aufgenommen werden können.

r Besondere Bedeutung hat die zuvor beschriebene Erfindung für die Entfernung von Thromben aus besonders kleinlumigen Gefäßen, insbesondere intrakraniellen Gefäßen. Selbstverständlich läßt sich die Erfindung jedoch auch bei der Entfernung von Thromben aus anderen Bereichen des Körpers einsetzen, beispielsweise aus dem Herzen, der Lunge, den Beinen etc. Denkbar ist auch der Einsatz zum Entfernen sonstiger Fremdkörper aus den Blutgefäßen, beispielsweise von Embolisationsspiralen und Stents.

Die Erfindung wird anhand der beigefügten Figuren näher erläutert, wobei nur die in Fig. 13 gezeigte Vorrichtung ein Ausführungsbeispiel der Erfindung ist, während die in den Figs. 1-12 gezeigten Vorrichtungen nicht in den Schutzbereich der Ansprüche fallen. Es zeigen:
- Figur 1: eine Darstellung einer Vorrichtung in der Seitenansicht;
- Figur 2 bis 6: eine Darstellung der Vorrichtung aus Figur 1 in verschiedenen Stadien der Entfernung eines Thrombus;
- Figur 7: die Vorrichtung gemäß einer alternativen Ausführungsform in der Seitenansicht;
- Figur 8: den Verlauf der Streben zum Aufbau einer Korbstruktur;
- Figuren 9a, b, c: einen alternativen Verlauf der Streben zum Aufbau einer Korbstruktur;
- Figur 10 a: die Verbindung von Streben zum Aufbau einer Korbstruktur im distalen Bereich durch Spiralhülsen;
- Figur 10b: die Korbstruktur aus Figur 10a im Querschnitt;
- Figur 11: die Vorrichtung gemäß einer weiteren Ausführungsform in der Seitenansicht;
- Figur 12: die Vorrichtung in einer weiteren Ausführungsform in der Seitenansicht und
- Figur 13: eine Ausführungsform der Erfindung.

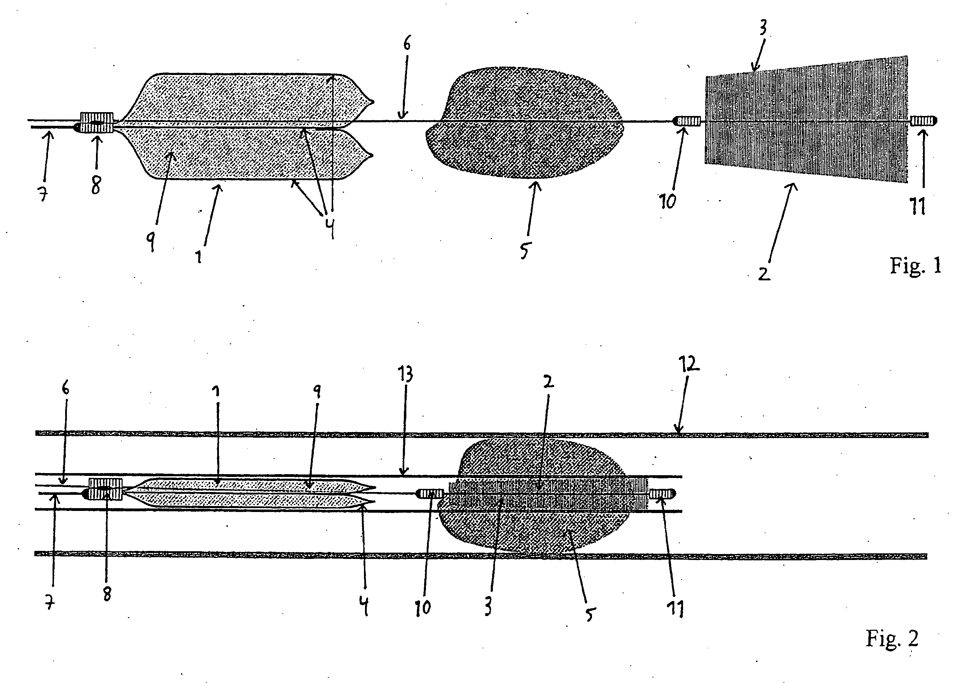

In Figur 1 ist eine erste Ausführungsform in der Seitenansicht dargestellt. Die Vorrichtung weist als wesentliche Bestandteile insbesondere eine Korbstruktur 1 sowie ein distales Element 2 auf. Vom distalen Element 2 stehen Fasern 3 radial nach außen hervor. Die Korbstruktur 1 setzt sich aus Streben 4 zusammen, die im Wesentlichen in Längsrichtung verlaufen. Die Vorrichtung dient dazu, zunächst mit Hilfe des distalen Elements 2 und den hiervon ausgehenden Fasern 3 einen Thrombus 5 durch Zurückbewegen des distalen Elements 2 in proximaler Richtung einzufangen und schließlich in die Korbstruktur 1 einzubringen. Bei den hier dargestellten Seitenansichten bedeutet proximal jeweils nach links, distal nach rechts. Distales Element 2 und Korbstruktur 1 sind über getrennte Führungsdrähte 6 bzw. 7 bewegbar. Dabei läuft die Korbstruktur 1 an ihrem proximalen Ende zentral in einer Hülse 8 zusammen, an der der Führungsdraht 7 für die Korbstruktur 1 festgelegt ist, während der Führungsdraht 6 für das distale Element 2 durch die Hülse 8 hindurch verläuft. Im weiteren Verlauf erstreckt sich der Führungsdraht 6 durch das Innere der Korbstruktur 1, was bewirkt, dass beim Zurückziehen des distalen Elements 2 dieses automatisch zusammen mit dem eingefangenen Thrombus 5 in die Korbstruktur 1 eingleitet. An ihrem distalen Ende ist die Korbstruktur 1 offen.

Entlang des radialen Umfanges ist die Korbstruktur 1 mit einer Polymerhaut 9 versehen, die dazu dient, einen eingefangenen Thrombus zusätzlich zu sichern. Distales Element 2 und Führungsdraht 6 sind über eine Mikrowendel 10 miteinander verbunden. Am distalen Ende weist die gesamte Vorrichtung eine distale Spitze 11 auf, die abgerundet ist und daher atraumatisch wirkt. Das distale Element 2 hat insgesamt eine konische Struktur, da die Fasern 3 in ihrer Länge von proximal nach distal zunehmen. Eine solche Ausführungsform hat den Vorteil, das unabhängig von der Weite des Blutgefäßes stets Fasern 3 vorhanden sind, die eine optimale Länge aufweisen. Darüber hinaus können die längeren Fasern 3 im distalen Bereich des distalen Elements 2 ggf. beim Zurückbewegen vom Thrombus 5 abfallende Thrombusfragmente einfangen.

In den Figuren 2 bis 6 ist die Vorrichtung aus Figur 1 im Einsatz dargestellt. In Figur 2 wird die Vorrichtung innerhalb eines Mikrokatheters 13 in ein Blutgefäß 12 eingebracht. Dabei sind sowohl Korbstruktur 1 als auch distales Element 2 stark komprimiert, wobei der Innendurchmesser des Mikrokatheters 13 die radiale Ausdehnung der Vorrichtung begrenzt. Der Mikrokatheter 13 wird seitlich am Thrombus 5 vorbeigeführt oder auch direkt durch den Thrombus 5 hindurch. Auf diese Weise wird erreicht, dass das distale Ende des Mikrokatheters 13 distal des Thrombus 5 liegt.

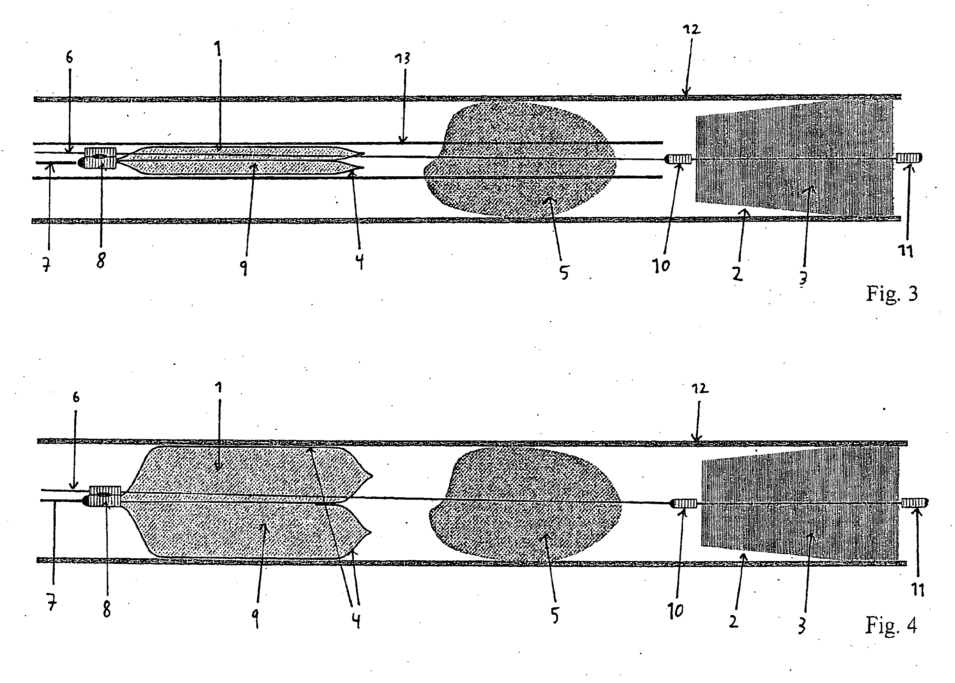

In Figur 3 ist das distale Element 2 aus dem Mikrokatheter 13 ausgeschoben worden und hat sich voll entfaltet, d. h. die Fasern 3 stehen nun deutlich weiter radial nach außen vor. Die Korbstruktur 1 hingegen befindet sich noch im Mikrokatheter 13 im komprimierten Zustand.

In Figur4 ist der Mikrokatheter 13 in proximaler Richtung zurückgezogen worden, so dass nun auch die Korbstruktur 1 aus dem Mikrokatheter 13 hervortreten konnte und ihre volle Korbstruktur angenommen hat. Der Außendurchmesser der Korbstruktur 1 entspricht nunmehr in etwa dem Innendurchmesser des Blutgefäßes 12. In der in den Figuren 2 bis 4 dargestellten Weise wird erreicht, dass das distale Element 2 distal des Thrombus 5 liegt, während die Korbstruktur 1 sich proximal des Thrombus 5 befindet.

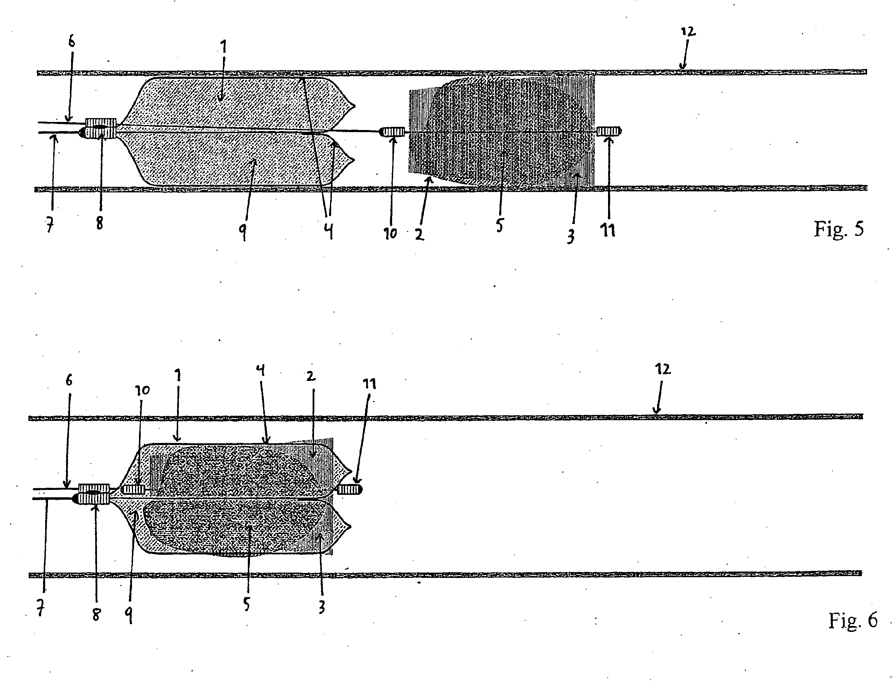

In Figur 5 ist dargestellt, wie der Thrombus 5 durch Zurückziehen in proximaler Richtung des distalen Elements 2 eingefangen wird. Dabei halten die Fasern 3 den Thrombus 5 fest und stabilisieren ihn, um zu verhindern, dass sich Thrombusfragmente abspalten und im Blutgefäßsystem verteilen.

In Figur 6 schließlich ist das distale Element 2 mitsamt des Thrombus 5 soweit zurückgezogen worden, dass es in die Korbstruktur 1 eingebracht ist. Selbstverständlich muß die Korbstruktur 1 am distalen Ende eine entsprechend große Öffnung aufweisen. Insgesamt wird der Thrombus 5 nun zusätzlich durch die Korbstruktur 1 mit der Polymerhaut 9 gesichert, so dass ein Verlorengehen des Thrombus 5 nicht mehr zu erwarten ist. Anschließend wird die gesamte Vorrichtung zurückgezogen, bis sie in einen Führungskatheter gelangt, der einen ausreichend großen Innendurchmesser aufweist, um die gesamte Vorrichtung aufzunehmen. Der Führungskatheter befindet sich in einem Blutgefäß 12 mit weiterem Durchmesser, wie auch bei dem hier dargestellten Blutgefäß 12 zu erkennen ist. Schließlich wird der Führungskatheter aus dem gesamten Blutgefäßsystem entfernt, der Thrombus 5 wurde restlos beseitigt.

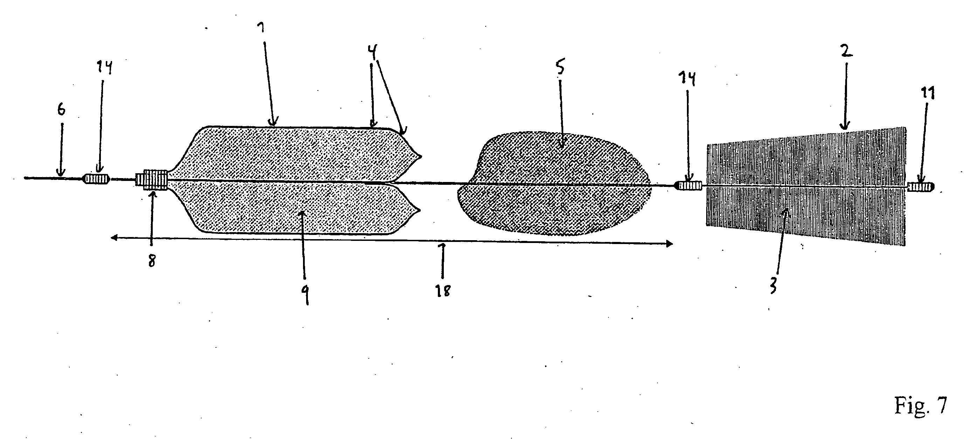

Figur 7 stellt eine alternative Ausführungsform mit lediglich einem Führungsdraht 6 für das distale Element auf. Die Korbstruktur 2 verfügt hier nicht über einen separaten Führungsdraht. Durch die Anschläge 14 auf dem Führungsdraht 6 wird hierbei jedoch sichergestellt, dass die auf dem Führungsdraht 6 längs verschiebliche Korbstruktur 1 lediglich zwischen diesen beiden Anschlägen 14 zu bewegen ist. Die Korbstruktur 1 kann somit lediglich in dem Bereich des Pfeiles 18 bewegt werden. Die Anschläge 14 sind so ausgebildet, dass ihr Durchmesser zu groß ist, als dass sie durch die Hülse 8 hindurch passen würden.

Beim Vorschub des gesamten Systems durch den Mikrokatheter wird auch die Korbstruktur durch den proximalen Anschlag 14 mit in distale Richtung geschoben. Nachdem dann das distale Element 2 distal des Thrombus 5 freigesetzt worden ist, wird der Mikrokatheter zurückgezogen, um auch die Korbstruktur 1 proximal des Thrombus 5 freizusetzen. In der Regel hält diese ihre axiale Position im Blutgefäß 12 aufgrund ihrer ausreichend hohen Radialkräfte. Sollte die Korbstruktur 1 ihre axiale Position nicht selbständig halten und sich in Richtung proximal bewegen, kann der Mikrokatheter 13 dazu verwendet werden, die Fixierung der Korbstruktur 1 zu unterstützen, indem der Mikrokatheter 13 entweder im Vorhinein nur so weit zurückgezogen wird, dass die Korbstruktur 1 sich vollständig entfalten kann, oder im Nachhinein wieder bis vor die Korbstruktur 1 geschoben wird.

Anschließend wird, wie zuvor beschrieben, das distale Element 2 zurückgezogen, um den Thrombus 5 einzufangen, bis schließlich distales Element 2 und Thrombus 5 in die Korbstruktur 1 gelangen. Bei weiterem Rückzug wird dann auch die Korbstruktur 1 mit in Richtung proximal bewegt, da der distal gelegene Anschlag 14 die Korbstruktur 1 mitnimmt. Schließlich kann die gesamte Vorrichtung weiter nach proximal bis in einen Führungskatheter zurückgezogen und entfernt werden.

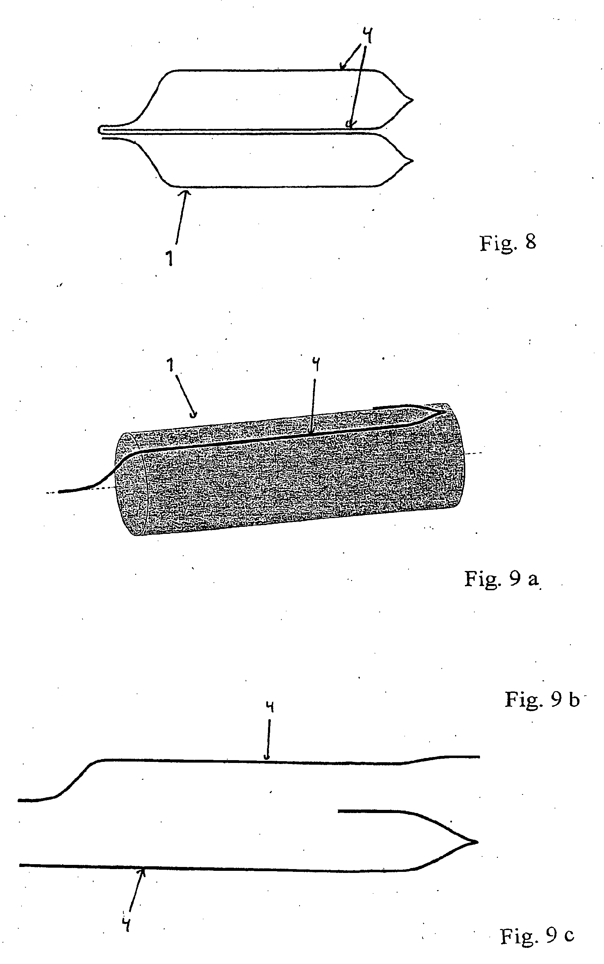

In Figur 8 ist der Aufbau einer Korbstruktur 1 aus Streben dargestellt, wobei in diesem Fall die Streben 4 aus einem in Schleifen gelegten Draht aufgebaut sind. Auf diese Weise erhält man nur wenige Drahtenden, die das Blutgefäß verletzen könnten. Darüber hinaus werden durch den gebogenen Draht nur zwei distale Kanten der Korbstruktur 1 gebildet, so dass die distale Öffnung der Korbstruktur 1 ausreichend groß ist. Ggf. können in die hier dargestellte Korbstruktur 1 noch zusätzliche Querstreben eingebracht werden, insbesondere im distalen Bereich, um die Korbstruktur 1 weiter zu stabilisieren und die Polymerhaut 9 zu halten.

In den Figuren 9a, 9b und 9c ist der mögliche Verlauf einer Strebe zum Aufbau einer Korbstruktur 1 dargestellt, wobei hier die Streben vom proximalen Ende der Korbstruktur 1 ausgehend nach radial außen verlaufen, sich in Längsrichtung nach distal erstrecken und in einer Schleife auf dem Umfang der Korbstruktur 1 partiell nach proximal zurücklaufen. Dabei stellt die Figur 9b eine Seitenansicht, die Figur 9c eine Draufsicht auf die Strebe 4 dar. Man erkennt, dass die Abknickung der Strebe 4 im proximalen Bereich senkrecht zur Abknickung der Strebe 4 im distalen Bereich erfolgt.

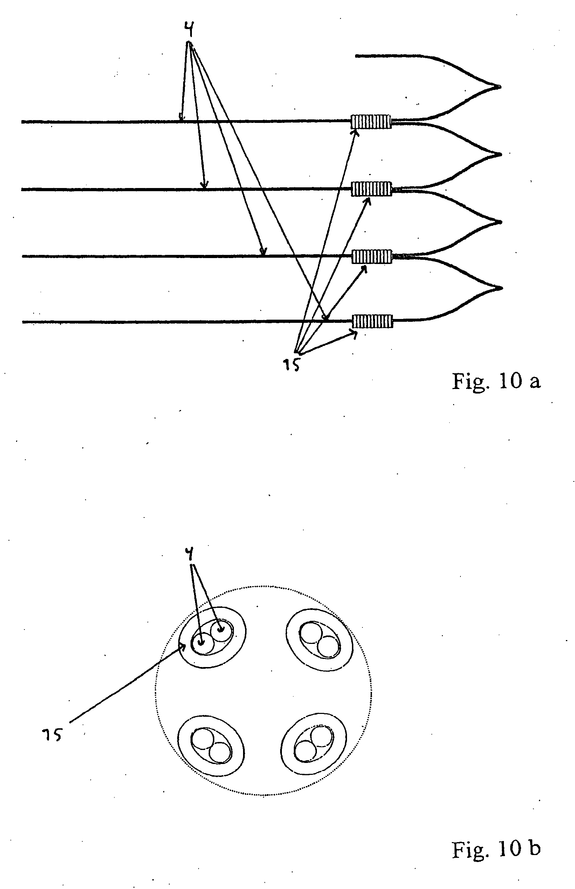

In Figur 10a ist dargestellt, wie die zurückgebogenen distalen Enden der Streben 4 mittels Spiralhülsen 15 miteinander verbunden sind. Hierbei ist zu beachten, dass in Figur 10a gewissermaßen eine auseinandergefaltete Korbstruktur 1 dargestellt ist. Tatsächlich verlaufen die Streben 4 selbstverständlich auf dem Umfang der Korbstruktur 1.

In Figur 10b ist ein Querschnitt der Darstellung aus Figur 10a dargestellt, bei der man erkennen kann, wie die insgesamt vier Spiralhülsen 15 jeweils zwei Streben 4 miteinander verbinden. Das Verbinden der Streben 4 kann zusätzlich oder auch alternativ zu den Spiralhülsen 15 mit Hilfe von Laserschweißpunkten erfolgen. Selbstverständlich ist es auch möglich, eine größere oder kleinere Anzahl an Streben 4 zu verwenden.

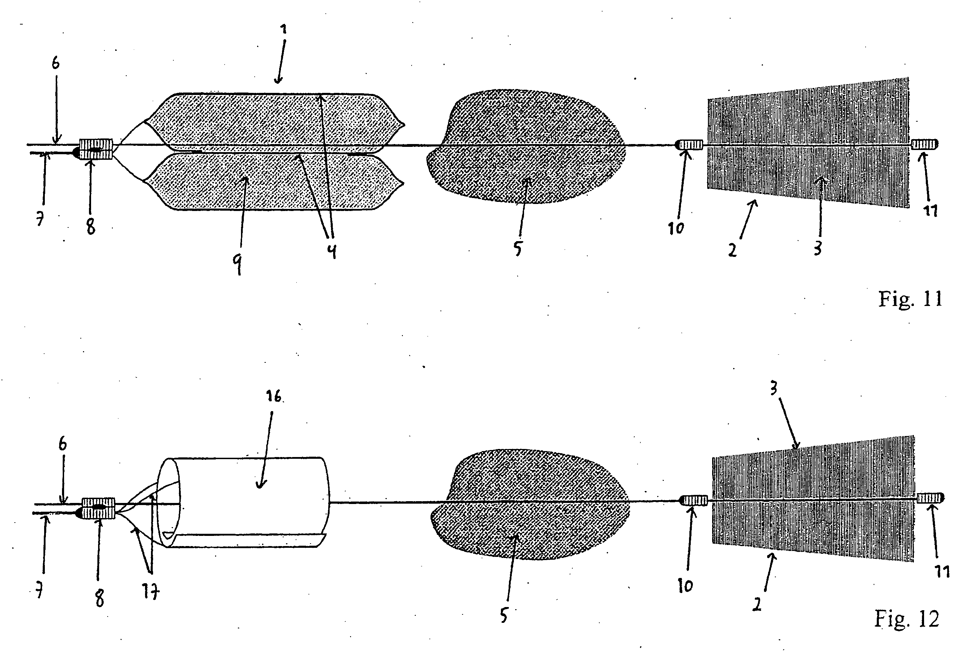

In Figur 11 ist eine alternative Ausführungsform dargestellt, bei der die Korbstruktur 1 an ihrem proximalen Ende nicht geschlossen ist. Dennoch wird eine ausreichende Sicherung des Thrombus 5 erreicht durch die Bespannung mit der Polymerhaut 9 entlang des radialen Umfanges der Korbstruktur 1. In diesem Fall hat die Korbstruktur 1 eher die Gestalt eines Schlauches oder einer Röhre als einer echten Korbstruktur.

In Figur 12 schließlich ist eine weitere Ausführungsform dargestellt, bei der anstelle einer Korbstruktur eine Röhrenstruktur 16 zum Einsatz kommt, die sich aus einem eingerollten Blech zusammensetzt. Dabei überlappen die seitlichen Enden des Bleches zu einem gewissen Maße, wobei die Röhrenstruktur 16 auch im expandierten Zustand maximal den Durchmesser annimmt, dass der seitliche Schlitz nicht offenliegt. Am proximalen Ende weist die Röhrenstruktur 16 Verbindungsstreben 17 auf, die dazu dienen, beim Zurückziehen eine Zusammenfaltung der Röhrenstruktur 16 zu gewährleisten. In der hier dargestellten Variante weist die Vorrichtung 2 getrennte Führungsdrähte 6, 7 auf, selbstverständlich ist jedoch auch eine Ausführungsform denkbar, bei der die Röhrenstruktur 16 mit lediglich einem Führungsdraht 6 kombiniert wird.

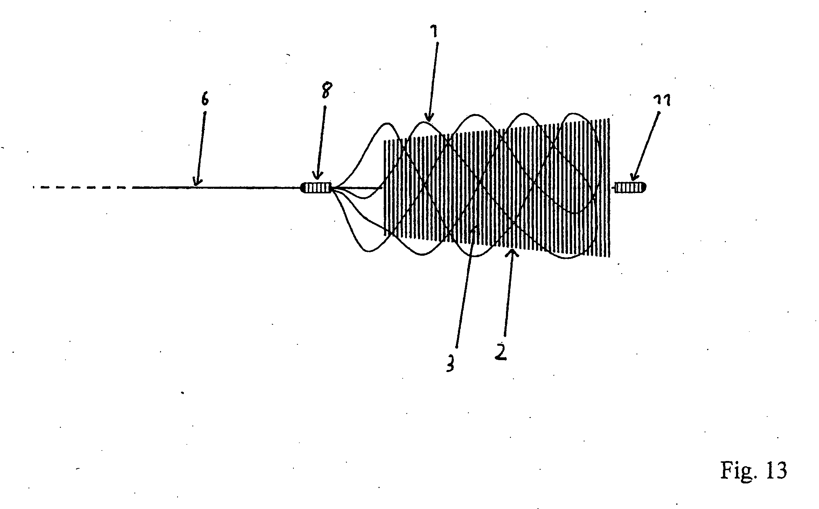

In Figur 13 ist die Ausführungsform der Erfindung dargestellt, bei der anstelle von gegeneinander längs verschieblicher Korbstruktur und distalem Element eine Drahtstruktur 1 vorgesehen ist, die sich nur teilweise über das distale Element 2 erstreckt. Die Drahtstruktur 1 ist als Geflecht ausgebildet, wobei die einzelnen Drähte in einer fest mit dem Führungsdraht 6 verbundenen Hülse auf dem Führungsdraht zusammenlaufen. Die Drahtstruktur 1 expandiert selbständig nach Ausschieben aus dem Mikrokatheter und ist aus Nitinol gefertigt. Die Gesamtzahl der Drähte kann dabei variieren, beispielhaft sind hier zwei Drähte dargestellt, die spiralförmig von proximal nach distal und zurück verlaufen und dabei ein Geflecht ausbilden. Selbstverständlich können jedoch auch drei oder mehr Drähte verwendet werden. Insgesamt zeichnet sich die hier dargestellte Ausführungsform der Erfindung durch eine kurze Bauform aus, die besonders in kleinen, gewinkelten Gefäßen Vorteile aufweist.

## Patentansprüche

1. Vorrichtung zur Entfernung von Fremdkörpern und Thromben (5) aus Körperhohlräumen und Blutgefäßen (12) mit mindestens einem Führungsdraht (6), der ein distales Element (2) in Form einer Mikrobürste aufweist, wobei das distale Element (2) radial nach außen hervorstehende Fasern (3) aufweist, **dadurch gekennzeichnet, dass** die Vorrichtung eine fest mit dem Führungsdraht (6) verbundene Drahtstruktur (1) in Form einer Korbstruktur aufweist, die geeignet ist, sich unter dem äußeren Zwang eines Mikrokatheters (13) eng zusammenzufalten und innerhalb des Mikrokatheters (13) transportiert und bei Wegfall des äußeren Zwangs durch den Mikrokatheter (13) zur vollen Drahtstruktur (1) entfaltet zu werden, wobei sich die Drahtstruktur (1) nur zum Teil über das distale Element (2) erstreckt

2. Vorrichtung nach Anspruch 1, **dadurch gekennzeichnet, dass** die Drahtstruktur (1) aus schlaufenförmig verlaufenden Drähten besteht.

3. Vorrichtung nach Anspruch 1 oder 2, **dadurch gekennzeichnet, dass** die Drahtstruktur (1) zumindest teilweise aus einem Formgedächtnismaterial, insbesondere Nitinol, besteht.

4. Vorrichtung nach einem der Ansprüche 1 bis 3, **dadurch gekennzeichnet, dass** die Fasern (3) eine Länge von 0,5 bis 6 mm und vorzugsweise 1,2 bis 3,0 mm aufweisen.

5. Vorrichtung nach einem der Ansprüche 1 bis 4, **dadurch gekennzeichnet, dass** die Fasern (3) spiralförmig entlang der Längsachse des distalen Elements (2) angeordnet sind.

6. Vorrichtung nach einem der Ansprüche 1 bis 5, **dadurch gekennzeichnet, dass** die Radialausdehnung der Fasern (3) des distalen Elements (2) von proximal nach distal zunimmt.

7. Vorrichtung nach einem der Ansprüche 1 bis 6, **dadurch gekennzeichnet, dass** die Fasern (3) im proximalen Bereich des distalen Elements (2) härter sind als im distalen Bereich des distalen Elements (2).

8. Vorrichtung nach einem der Ansprüche 1 bis 7, **dadurch gekennzeichnet, dass** die Fasern (3) einen Winkel von 70° bis 110°, vorzugsweise einen Winkel von 80° bis 90°, zur Längsachse der Vorrichtung ausbilden.

9. Vorrichtung nach einem der Ansprüche 1 bis 8, **dadurch gekennzeichnet, dass** die Fasern (3) durch Verflechten, Einklemmen, Kleben, Verknoten, Verschweißen und/oder Anschmelzen am distalen Element (2) festgelegt sind.

10. Vorrichtung nach einem der Ansprüche 1 bis 9, **dadurch gekennzeichnet, dass** die radial außen liegenden Enden der Fasern (3) Verdickungen aufweisen.

11. Vorrichtung nach einem der Ansprüche 1 bis 10, **dadurch gekennzeichnet, dass** die radial außen liegenden Enden der Fasern (3) zumindest teilweise über Schlaufen miteinander verbunden sind.

12. Vorrichtung nach einem der Ansprüche 1 bis 11, **dadurch gekennzeichnet, dass** die Fasern (3) zumindest teilweise unterschiedlich weit zu den Seiten des distalen Elements (2) radial nach außen hervorstehen.

13. Vorrichtung nach einem der Ansprüche 1 bis 12, **dadurch gekennzeichnet, dass** die Fasern (3) mit einer Beschichtung versehen sind.

14. Vorrichtung nach einem der Ansprüche 1 bis 13, **gekennzeichnet durch** einen oder mehrere radiopake Marker.

15. Vorrichtung nach einem der Ansprüche 1 bis 14, in Kombination mit einem Führungskatheter und/oder Mikrokatheter (13), wobei der Führungs- oder Mikrokatheter (13) ein Aspirationskatheter sein kann.

## Claims

1. Device for the removal of foreign objects and thrombi (5) from body cavities and blood vessels (12) using at least one guide wire (6) provided with a distal element (2) in form of a micro-brush, wherein the distal element (2) is provided with fibers (3) projecting radially outward **characterized in that** the device is provided with a wire structure (1) that is permanently connected with the guide wire (6) and has a cage structure, which is suitable to be flatly collapsible under the external strain exerted by a micro-catheter (13) and to be transported inside the micro-catheter (13) and to unfold to its full wire structure (1) when the external constraint exerted by the micro-catheter (13) is removed, with the wire structure (1) extending only partially over the distal element (2).

2. Device according to claim 1, **characterized in that** the wire structure (1) consists of wires extending in a loop-shaped fashion.

3. Device according to claim 1 or 2, **characterized in that** the wire structure (1) consists, at least partially, of a shape-memory material, in particular nitinol.

4. Device according to any of claims 1 to 3, **characterized in that** the fibers (3) have a length of between 0.5 and 6 mm and preferably between 1.2 and 3.0 mm.

5. Device according to any of claims 1 to 4, **characterized in that** the fibers (3) are arranged spirally along the longitudinal axis of the distal element (2).

6. Device according to any of claims 1 to 5, **characterized in that** the radial extension of the fibers (3) of the distal element (2) increases from proximal to distal.

7. Device according to any of claims 1 to 6, **characterized in that** the fibers (3) in the proximal area of the distal element (2) are harder than in the distal area of the distal element (2).

8. Device according to any of claims 1 to 7, **characterized in that** the fibers (3) form an angle with the longitudinal axis of the device that ranges between 70° and 110°, preferably between 80° and 90°.

9. Device according to any of claims 1 to 8, **characterized in that** the fibers (3) are secured or attached to the distal element (2) by braiding, clamping, bonding, knotting, welding and/or fusing.

10. Device according to any of claims 1 to 9, **characterized in that** the ends of the fibers (3) located radially outward are provided with slubs.

11. Device according to any of claims 1 to 10, **characterized in that** the ends of the fibers (3) located radially outward are at least in part connected with each other by means of loops.

12. Device according to any of claims 1 to 11, **characterized in that** the fibers (3), at least partially, protrude differently far radially outward at the sides of the distal element (2).

13. Device according to any of claims 1 to 12, **characterized in that** the fibers (3) are provided with a coating.

14. Device according to any of claims 1 to 13, **characterized by** one or several radiopaque markers.

15. Device according to any of claims 1 to 14 in combination with a guide catheter and/or micro-catheter (13), wherein the guide or micro-catheter (13) may be designed as an aspiration catheter.

## Revendications

1. Dispositif pour l'extraction de corps étrangers et de thrombus (5) à partir de cavités corporelles et de vaisseaux sanguins (12), comprenant au moins un fil de guidage (6) qui présente un élément distal (2) en forme de micro-brosse, étant entendu que l'élément distal (2) présente des fibres (3) faisant saillie radialement vers l'extérieur, **caractérisé en ce que** le dispositif présente une structure en fil (1) en forme de structure de panier reliée de façon fixe avec le fil de guidage (6) qui est conçue pour se replier étroitement sous la contrainte extérieure d'un micro-cathéter (13) et pour être transportée à l'intérieur du micro-cathéter (13) et déployée au travers du micro-cathéter (13), formant une structure en fil (1) complète, lorsque la contrainte extérieure est relâchée, étant entendu que la structure en fil (1) ne s'étend qu'en partie au-delà de l'élément distal (2).

2. Dispositif selon la revendication 1, **caractérisé en ce que** la structure en fil (1) est constituée de fils s'étendant en forme de boucles.

3. Dispositif selon la revendication 1 ou 2, **caractérisé en ce que** la structure en fil (1) est constituée au moins partiellement d'un matériau à mémoire de forme, en particulier le Nitinol.

4. Dispositif selon l'une des revendications 1 à 3, **caractérisé en ce que** les fibres (3) présentent une longueur de 0,5 à 6 mm, et de préférence de 1,2 à 3,0 mm.

5. Dispositif selon l'une des revendications 1 à 4, **caractérisé en ce que** les fibres (3) sont agencées en forme de spirale le long de l'axe longitudinal de l'élément distal (2).

6. Dispositif selon l'une des revendications 1 à 5, **caractérisé en ce que** l'extension radiale des fibres (3) de l'élément distal (2) augmente du point proximal vers le point distal.

7. Dispositif selon l'une des revendications 1 à 6, **caractérisé en ce que** les fibres (3) sont plus dures dans la zone proximale de l'élément distal (2) que dans la zone distale de l'élément distal (2).

8. Dispositif selon l'une des revendications 1 à 7, **caractérisé en ce que** les fibres (3) forment un angle de 70° à 110°, et de préférence un angle de 80° à 90°, par rapport à l'axe longitudinal du dispositif.

9. Dispositif selon l'une des revendications 1 à 8, **caractérisé en ce que** les fibres (3) sont fixées sur l'élément distal (2) par maillage, intersection, collage, nouage, soudage et/ou fusion.

10. Dispositif selon l'une des revendications 1 à 9, **caractérisé en ce que** les extrémités des fibres (3) situées vers l'extérieur dans la direction radiale présentent des épaississements.

11. Dispositif selon l'une des revendications 1 à 10, **caractérisé en ce que** les extrémités des fibres (3) situées vers l'extérieur dans la direction radiale sont reliées les unes avec les autres au moins partiellement au moyen de boucles.

12. Dispositif selon l'une des revendications 1 à 11, **caractérisé en ce que** les fibres (3) font saillie radialement vers l'extérieur au moins partiellement à des distances différentes par rapport aux côtés de l'élément distal (2).

13. Dispositif selon l'une des revendications 1 à 12, **caractérisé en ce que** les fibres (3) sont munies d'un revêtement.

14. Dispositif selon l'une des revendications 1 à 13, **caractérisé par** un ou plusieurs marqueurs opaques aux radiations.

15. Dispositif selon l'une des revendications 1 à 14, en combinaison avec un cathéter de guidage et/ou un micro-cathéter (13), étant entendu que le cathéter de guidage et/ou le micro-cathéter (13) peut être un cathéter d'aspiration.
